Europäisches Patentamt

European Patent Office

Office européen des brevets

(11) Publication number: **0 448 770 A1**

# EUROPEAN PATENT APPLICATION

(21) Application number: 90113376.9

(22) Date of filing: 12.07.90

(51) Int. Cl.⁵: **C07K 15/00**, C08H 1/06, A61L 27/00

(30) Priority: 28.03.90 JP 79782/90
05.04.90 JP 90767/90
08.05.90 JP 118016/90

(43) Date of publication of application:
02.10.91 Bulletin 91/40

(84) Designated Contracting States:
CH DE FR GB IT LI

(71) Applicant: Takasu, Katsuya
124-1, Aza-kamigochu, Oaza-akabane,
Ishiki-cho
Hazu-gun, Aichi-ken(JP)

(72) Inventor: Takasu, Katsuya
124-1, Aza-kamigochu, Oaza-akabane,
Ishiki-cho
Hazu-gun, Aichi-ken(JP)
Inventor: Fournier, Pierre F.
57 Avenue De Villiers
F-75017 Paris(FR)

(74) Representative: Liedl, Gerhard, Dipl.-Phys.
Patentanwalt Liedl Steinsdorfstrasse 21-22
W-8000 München 22(DE)

(54) Method for separating collagen.

(57) A method for separating collagen including adding distilled water to a piece of fat which is extracted from a human body, so that the fat cells are destroyed by the osmotic pressure of the distilled water, and separating collagen from a fat component which has a smaller specific gravity than that of the collagen.

FIG-1

The present invention relates to a method for separating collagen, and more precisely, it relates to a method for separating collagen from fat of a human body.

In cosmetic surgery, it is known to inject collagen into a patient's skin to remove or remedy senile lines (wrinkles) or depressions, such as a scar. Collagen which is a main component of a connective tissue which constitutes bones and skin, etc. is made of fibrous protein. Accordingly, the skin with depressions or the wrinkled skin can be flattened or remedied by the collagen which is directly injected into the skin with an injector.

Collagen which is used in such conventional cosmetic surgery is usually extracted and refined from cowhide. However, since the collagen is a heterogeneous protein from a human body, there is a possibility of an allergic reaction.

The inventor of the present invention has focused on a technology of fat extraction from a human body which is widely used in a cosmetic surgery. Namely, the inventor has conceived a separation of collagen from the extracted fat. In case of collagen which is separated from his or her own fat, no allergic reaction occurs , unlike the prior art in which an allergic reaction occurs owing to a hetelogeneous protein, as mentioned above.

The primary object of the present invention is to provide a method for effectively and simply separating collagen from fat of a human body.

To achieve the object mentioned above, four different collagen separation methods are provided according to the present invention. These are a first separation method by distilled water, a second separation method by freezing, a third separation method by filtering, and a fourth separation method by ultrasonic oscillation.

According to a first aspect (first separation method) of the present invention, there is provided a method for separating collagen comprising adding distilled water to a piece of fat which is extracted from a human body, so that the fat cell is destroyed by the osmotic pressure of the distilled water, and separating collagen from a fat component which has a smaller specific gravity than that of the collagen.

Preferably, the separation of the collagen from the fat component is effected by a centrifugal separation.

According to a second aspect (second separation method) of the present invention, there is provided a method for separating collagen comprising adding distilled water to a piece of fat which is extracted from a human body, quickly freezing the mixture of the fat piece and the distilled water to a temperature below -50°C to destroy the fat cells, and then separating collagen from a fat component which has a smaller specific gravity than that of the collagen.

According to a third aspect (third separation method) of the present invention, there is provided a method for separating collagen comprising adding a small amount of distilled water to a piece of fat which is extracted from a human body, and filtering the mixture of the fat piece and the distilled water to separate collagen from the fat cells.

Preferably, the separation of the collagen from the fat cells is effected by a filter which has a large number of openings of 100-500 $\mu$m diameter.

According to a fourth aspect (fourth separation method) of the present invention, there is provided a method for separating collagen comprising vibrating a piece of fat which is extracted from a human body with an ultrasonic vibrator to destroy the fat cells, and separating collagen from a fat component which has a smaller specific gravity than that of the collagen.

Preferably, the ultrasonic vibration is effected by an electrical ultrasonic vibrator.

This invention will be described below with reference to the accompanying drawings, in which;

Fig.1 is a enlarged plan view of a filter which is used in the third separation method according to the present invention; and

Fig.2 is a schematic view of an ultrasonic vibrator which is used in the fourth separation method according to the present invention.

Preferred enbodiments of the invention are as follows.

First Separation Method

In the first separation method, distilled water is used to separate collagen from fat.

Namely, a piece of fat which is extracted from a human body by a known surgical means is pulverized into gruel form by a stirrer (homogenizer). Thereafter, distilled water is added to the gruel-like pulverized fat piece. A fat cell is composed by an outer shell which is mainly made of collagen and a fat component surrounded by the outer shell. The addition of distilled water causes the fat cell to be completely destroyed by the osmotic pressure, so that the collagen and the fat component are separated from one another. In this state, however, the fat cells including the collagen and the fat components look like orange juice.

Thereafter, the liquid component is introduced into a centrifugal separator in which the liquid component is divided into a jellied collagen having a large specific gravity and an oily fat component having a small specific gravity. The separated collagen is taken out by an autopipette or the like.

The collagen (autocollagen) obtained by this method can be directly used as a collagen to be injected into the person himself or herself. It is also possible to freeze the collagen for a long time storage. The frozen collagen can be defrosted to be used.

Second Separation Method

In this method, the collagen is separated from fat by a quick freezing.

Namely, a piece of fat which is extracted from a human body by a known surgical means, similar to the first separation method, is mixed with distilled water, so that the fat cells expand owing to the osmotic pressure of the distilled water.

Thereafter, the fat cells are quickly frozen to a temperature below -50° C. Consequently, the water component of the fat cells is frozen, so that a further expansion of the fat cells takes place, thus resulting in a complete destruction of the fat cells.

When the frozen fat cells are defrosted, a liquid product (semi-product) including the collagen and the fat component mixed therewith can be obtained.

Thereafter, the liquid semi-product is divided into a jellied collagen having a large specific gravity and an oily fat component having a small specific gravity by a centrifugal separator. The separated collagen is taken out by an autopipette or the like.

In this method, the frozen fat cells which are obtained in the freezing process mentioned above can be stored for a long time and can be defrosted and separated into the collagen and the fat component when used.

Third Separation Method

In the third method, the collagen is separated from fat by filtering.

Namely, a piece of fat is extracted from a human body by a known surgical means, so that a small quantity of distilled water is added to the extracted fat piece. The addition of distilled water causes a fat cell which is composed of an outer shell which is mainly made of collagen and a fat component surrounded by the outer shell, as mentioned before, to be completely destroyed by the osmotic pressure, so that the collagen and the fat component are separated from one another. In this state, however, the fat cells including the collagen and the fat component look like orange juice. Preferably, the quantity of distilled water is about 20% relative to the amount of the fat cells.

In the third embodiment, the fat is extracted into a fat extracting injector (10 cm³) in which about 2 cm³ of distilled water is contained in advance.

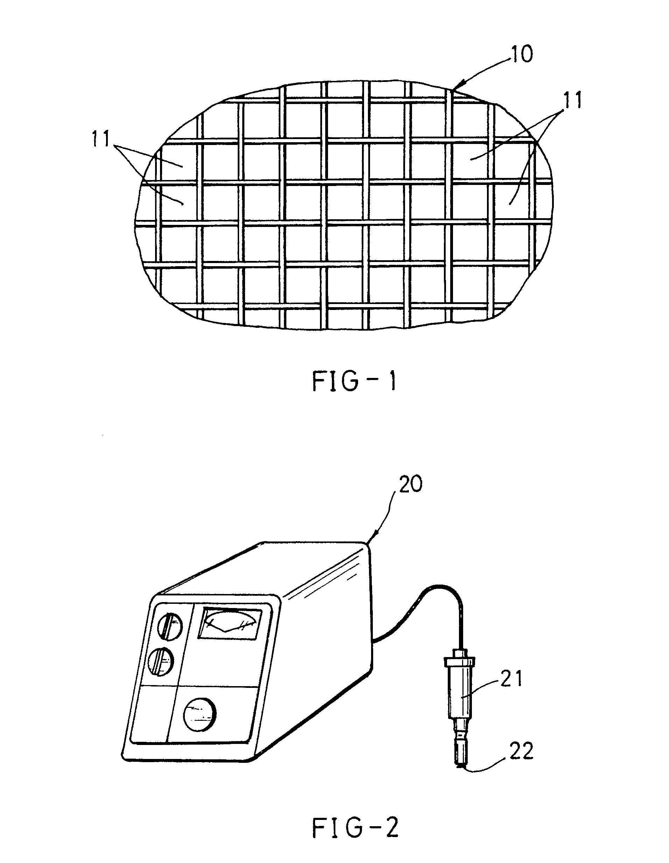

Thereafter, the liquid semi-product including the collagen and the fat component is filtered to quickly separate a fibered and jellied collagen and an oily fat component. As shown in Fig. 1, the size of the openings 11 of the filter 10 used in this method is such that the fibered collagen can be effectively separated, and preferably is 100-500 μm . A filter to be advantageously used is a polyester filtering net which is usually used in a blood transfusion instrument and which has about 100 μm diameter of net wires and about 200 μm diameter of the openings.

Upon filtering, a slight hydraulic pressure is preferably applied. In the embodiment, the fat component having distilled water mixed therewith is delivered with pressure from the injector.

The collagen which is separated and maintained in the filter is taken out.

The collagen (autocollagen) obtained by the third separation method can be directly used as collagen to be injected into the person himself or herself. It is also possible to freeze the collagen for a long time storage. The frozen collagen can be defrosted to be used.

Fourth Separation Method

In the fourth method the collagen is separated from fat by ultrasonic vibration.

Namely, a piece of fat which is extracted from a human body by a known surgical means is introduced into an electrical ultrasonic generator. As the electrical ultrasonic generator used in this embodiment can be used an ultrasonic cell crushing device, such as a "SONIFIER" which is available on the market by Branson Co. Ltd. in the United States which is used to destroy or crush an animal cell or plant tissue, to destroy bacteria or yeast, to cut DNA chain or to separate virus of DNA cells, etc.

As shown in Fig.2, the ultrasonic cell crushing device 20 has a converter which converts electrical energy to longitudinal mechanical vibration. When a tip 22 of the front end of a horn 21 of the device 20 is put in a solution, the mechanical vibration is transmitted to the solution as a pressure wave to cause cavitation. Namely, the tip of the horn put in the fat solution produces a pressure wave in the solution, so that air bubbles are produced by local negative pressure at the negative cycle of pressure wave. The air bubbles are pressed and collapsed at the positive cycle of pressure wave (This is called cavitation). The fat cells are destroyed by the strong impact of the repeated formation and destruction of the air bubbles in the solution.

The fat cells are thus completely destroyed by the ultrasonic vibration, so that the oil (fat) component and the collagen are separated from one another. In this state, however, the fat cells including the collagen and the fat component look like orange juice.

Thereafter, the liquid component is introduced into a centrifugal separator in which the liquid component is divided into a jellied collagen having a large specific gravity and an oily fat component having a small specific gravity. The separated collagen is taken out by an autopipette or the like.

The collagen (autocollagen) obtained by this method can be directly used as a collagen to be injected into the person himself or herself. It is also possible to freeze the collagen for a long time storage. The frozen collagen can be defrosted to be used.

As can be understood from the foregoing, according to the present invention, the collagen which is extracted from a patient himself or herself can be injected as so called "autocollagen" into his or her own body. Namely, the wrinkled skin or the skin with depressions, such as a scar, or depressions caused by pimple can be flattened or remedied by the collagen which is directly injected into a patient's dermis of the senile lines (wrinkles) or depressions by an injector without an allergic reaction. The removal of such senile lines causes the patient to expect to have a rejuvenated skin.

Since the collagen has a moisture effect in which an oil component and moisture of a skin is maintained on the skin surface, the collagen can be used as a cosmetic cream, a cosmetic latex, or other cosmetics to maintain the moisture of the skin. In this case, the autocollagen obtained by the present invention can be used as a high quality cosmetic product for himself or herself. As mentioned before, in case of an autocollagen, no allergic reaction occurs.

As can be seen from the above discussion, according to the present invention, it is possible to easily and effectively separate collagen from fat. The separated collagen, if frozen, can be stored for a long period and can be used anytime by defrosting the same.

## Claims

1. A method for separating collagen comprising;
   adding distilled water to a piece of fat which is extracted from a human body, so that the fat cells are destroyed by the osmotic pressure of the distilled water; and, separating collagen from a fat component which has a smaller specific gravity than that of the collagen.

2. A separation method according to claim 1, wherein the separation of the collagen from the fat component is effected by a centrifugal separation.

3. A method for separating collagen comprising;
   adding distilled water to a piece of fat which is extracted from a human body; quickly freezing the mixture of the fat piece and the distilled water to a temperature below -50°c to destroy the fat cells; and thereafter, separating collagen from a fat component which has a smaller specific gravity than that of the collagen.

4. A separation method according to claim 3, wherein the separation of the collagen from the fat component is effected by a centrifugal separation.

5. A method for separating collagen comprising;
   adding a small amount of distilled water to a piece of fat which is extracted from a human body; and, filtering the mixture of the fat piece and the distilled water to separate collagen from the fat cells.

6. A separation method according to claim 5, wherein the separation of the collagen from the fat cells is effected by a filter which has a large number of openings of 100-500 $\mu$m diameter.

7. A method for separating collagen comprising;
   vibrating a piece of fat which is extracted from a human body by an ultrasonic vibrator to destroy the fat cells; and, separating collagen from a fat component which has a smaller specific gravity than that of the collagen.

8. A separation method according to claim 7, wherein the ultrasonic vibration is effected by an electrical ultrasonic vibrator which converts electrical energy to a mechanical ultrasonic vibration.

9. A separation method according to claim 7, wherein the separation of the collagen from the fat component is effected by a centrifugal separation.

FIG-1

FIG-2

European
Patent Office

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | EP-A-0 284 789  (ISTITUTO GENTILI) <br> * Whole document, especially example 1; column 4, lines 25-55 * <br> – – – | 1-6 | C 07 K 15/00 <br> C 08 H 1/06 <br> A 61 L 27/00 |
| A | EP-A-0 330 389  (KELMAN) <br> * Example 1; page 11, lines 25-46 * <br> – – – | 1-6 | |
| A | DE-A-1 936 957  (SCHALLER et al.) <br> * Page 2, lines 6-33 * <br> – – – | 1-6 | |
| A | CHEMICAL ABSTRACTS, vol. 74, no. 7, 15th February 1971, page 157, abstract no. 29867b, Columbus, Ohio, US; M.G. STEELE et al.: "Age changes in the subunit composition of rat tail tendon collagen extracted at 65 degrees in water and at 2 degrees in acid", <br> & GERONTOLOGIA 1970, 16(5), 277-82 <br> * Abstract * <br> – – – | 1-6 | |
| E | EP-A-0 418 979  (ZOCCHI) <br> * Whole document * <br> – – – – – | 7-9 | |
| | | | **TECHNICAL FIELDS SEARCHED (Int. Cl.5)** <br><br> C 07 K <br> C 08 H <br> A 61 L |

The present search report has been drawn up for all claims

| Place of search | Date of completion of search | Examiner |
|---|---|---|
| The Hague | 06 July 91 | MASTURZO P. |

CATEGORY OF CITED DOCUMENTS

X : particularly relevant if taken alone
Y : particularly relevant if combined with another
     document of the same category
A : technological background
O : non-written disclosure
P : intermediate document
T : theory or principle underlying the invention

E : earlier patent document, but published on, or after
     the filing date
D : document cited in the application
L : document cited for other reasons
.....................................................................................
& : member of the same patent family, corresponding
     document